# EUROPEAN PATENT APPLICATION

(11) **EP 4 843 562 A1**
(43) Date of publication of application: **25.11.2026**
(21) Application number: 25177782.7
(22) Date of filing: 20.05.2025
(51) Int. Cl.: A61K 9/00, A61K 9/08, A61K 31/472, A61K 47/40, A61P 27/02, A61P 27/04, A61P 27/06

(54) **AQUEOUS OPHTHALMIC COMPOSITION OF A LOW WATER-SOLUBLE TRPV1 ANTAGONIST**

(71) Applicant: Bausch + Lomb Ireland Limited, Dublin D24 PPT3 (IE)
(72) Inventor: VOIGT, Mirko, 16540 Hohen Neuendorf (DE); KOUTSAMANIS, loannis, 12157 Berlin (DE); KRÖHNE, Lutz, 16552 Schildow (DE)
(74) Representative: Maiwald GmbH

(57) **Abstract**

The present invention concerns an aqueous ophthalmic composition comprising 1-(6-fluoro-5-((((1r,3r)-3-(4-fluoro-3 (trifluoromethyl) phenoxy)-cyclobutyl)-amino)methylisoquinolin-8-yl)ethane-1.2-diol (Compound I), and a cyclodextrin, wherein the molar ratio of the cyclodextrin to the Compound I is from 5:1 to 19:1.

## Description

The present invention relates to an aqueous ophthalmic composition comprising 1-(6-fluoro-5-((((1r,3r)-3-(4-fluoro-3-(trifluoromethyl)phenoxy)cyclobutyl)amino)methyl)isoquinolin-8-yl)ethane-1,2-diol (Compound I), methods for producing the same and the use thereof.

Patients suffering from ocular surface pain, particularly chronic ocular surface pain, have a significant decline in quality of life, and many develop depression and in some cases become suicidal. In many patients, the ocular surface pain remains unresolved despite treatment of the underlying pathology (e.g., recent trauma or surgery, infection, or inflammation) and other known treatments cannot be used for long term therapy.

The Transient Receptor Potential Vanilloid 1 (TRPV1) receptor is a key receptor involved in the sensation of pain and, consequently, also in ocular surface pain. Therefore, the significance and potential benefits of TRPV1 antagonists for the treatment of ocular surface pain are subjects of current intensive research. WO 2024/062389 A1 describes polymorphs and formulations of the TRPV1 antagonist 1-(6-fluoro-5-((((1r,3r)-3-(4-fluoro-3-(trifluoromethyl)phenoxy)cyclobutyl)amino)-methyl)isoquinolin-8-yl)ethane-1,2-diol (Compound I) which may be suitable for the treatment of ocular surface disorders.

Eye drops are widely accepted by patients and their ease of use contributes to good patient compliance. The vast majority of marketed ocular formulations therefore exist in liquid state. In addition, an active pharmaceutical ingredient present in the dissolved state might show increased bioavailability and it can be accurately dosed compared to, e.g., suspensions, which results in lower side effects.

However, the solubility of Compound I in aqueous media and at pH values relevant for ocular administration (i.e., pH 6.0 - 8.0) is very low and may be in the range from 0.06 to 0.150 mg/mL. Emulsions or the addition of organic solvents can overcome this problem but have their own drawbacks. Emulsions or nanoemulsions require more sophisticated manufacturing methodologies, scale-up is not trivial and shelf-life stability can be challenging. Moreover, most organic solvents are unacceptable for ocular drug delivery systems or may cause ocular irritations.

In view of the foregoing, there is an ongoing need in the art for providing an aqueous ophthalmic formulation of a TRPV1 antagonist, which avoids the know disadvantages.

Accordingly, it is an object of the present invention, to provide an aqueous ophthalmic formulation, which contains a TRPV1 antagonist in a therapeutically effective quantity and is well-tolerable. It is desirable that the formulation can be prepared with standard manufacturing methodologies and is storage stable.

Moreover, it is an object of the present invention to provide an aqueous ophthalmic formulation for use as a medicament. It is desirable that the composition is suitable for treating diseases or conditions of the eye, or the organs or tissues surrounding the eye, or being connected herewith, and preferably for treating ocular surface disorders.

The above-mentioned and further objects are solved according to the subject-matter as defined in the independent claims.

According to a first aspect of the present invention, an aqueous ophthalmic composition is provided comprising: 1-(6-fluoro-5-((((1r,3r)-3-(4-fluoro-3(trifluoromethyl)phenoxy)-cyclobutyl)-amino)methyl)isoquinolin-8-yl)ethane-1.2-diol (Compound I) having structure 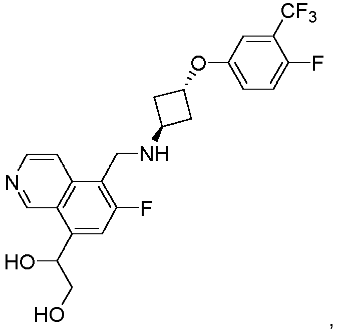 and
a cyclodextrin,
wherein the molar ratio of the cyclodextrin to the Compound I is from 5:1 to 19:1.

According to a further aspect, a method for preparing an aqueous ophthalmic composition according to the present invention is provided, wherein the method comprises the steps of:
mixing the Compound I, the cyclodextrin, and water, and
adjusting the pH to a range from 5.0 to 8.0.

According to still a further aspect, an aqueous ophthalmic composition according to the present invention for use as a medicament is provided.

According to still a further aspect, an aqueous ophthalmic composition according to the present invention for use in treating a disease or a condition of the eye or the organ or tissue surrounding the eye or being connected therewith is provided,
preferably for use in treating ocular surface disorders,
more preferably for use in treating ocular surface pain, dry eye disease, Sjogren's Syndrome, conjunctivitis, Map-Dot-Fingerprint Dystrophy, acanthamoeba, fibromyalgia, Meibomian gland dysfunction, thyroid eye disease, rosacea, ptosis, keratoconus, ocular pain syndrome, Steven-Johnson's syndrome, corneal epitheliopathies, corneal neuropathies, corneal dystrophies, epithelial basement membrane dystrophy, corneal erosions or abrasions, ocular surface diseases, blepharitis, graft vs. host disease, meibomitis, glaucoma, conjunctivochalasis, keratopathis, keratitis, iritis, episclentis, corneal surgery, multiple sclerosis, trichiasis, pterygium, neuralgia, xerophthalmia, or patients recovering from neurotrophic keratitis, and
most preferably for use in treating ocular surface pain.

According to still a further aspect, a container comprising the aqueous ophthalmic composition according to the present invention is provided, wherein the container is a single-dose container, preferably a single dose ophtiole, or a multi-dose container, preferably an ophtiole, or an application system, preferably a tip-seal application system, a pump-based application system, a standard bottle/dropper system, or a multi-dose application system.

Further preferred embodiments are described in the corresponding sub-claims.

According to one embodiment the cyclodextrin is selected from the group consisting of α-cyclodextrin, β-cyclodextrin, γ-cyclodextrins, derivatives thereof, and mixtures thereof, preferably the cyclodextrin is selected from the group consisting of α-cyclodextrin, β-cyclodextrin, methyl-β-cyclodextrin, 2-hydroxypropyl-β-cyclodextrin, 2-hydroxyethyl-β-cyclodextrin, sulfobutylether-β-cyclodextrin, heptakis (2,6-di-*O*-methyl)-β-cyclodextrin, heptakis (2,3,6-tri-*O*-methyl)-β-cyclodextrin, succinyl- β-cyclodextrin, 6-*O*-α-Maltosyl- β-cyclodextrin, 2-hydroxypropyl-γ-cyclodeotrin, 2-hydroxyethyl-γ-cyclodextrin, and mixtures thereof, more preferably the cyclodextrin is selected from the group consisting of β-cyclodextrin, , methyl-β-cyclodextrin, 2-hydroxypropyl-β-cyclodextrin, 2-hydroxyethyl-β-cyclodextrin, sulfobutylether-β-cyclodextrin, heptakis (2,6-di-*O*-methyl)-β-cyclodextrin, heptakis (2, 3, 6-tri-*O*-methyl)-β-cyclodextrin, succinyl- β-cyclodextrin, 6-*O*-α-Maltosyl- β-cyclodextrin, and mixtures thereof, and most preferably the cyclodextrin is 2-hydroxypropyl-β-cyclodextrin.

According to a further embodiment the composition comprises: the Compound I in an amount from 0.01 to 1 wt.-%, preferably from 0.02 to 0.7 wt.-%, more preferably from 0.04 to 0.5 wt.-%, and most preferably from 0.06 to 0.3 wt.-%, based on the total weight of the composition, and/or the cyclodextrin in an amount from 0.01 to 50 wt.-%, preferably from 0.05 to 40 wt.-%, more preferably from 0.07 to 30 wt.-%, and most preferably from 0.1 to 20 wt.-%, based on the total weight of the composition, and/or wherein the molar ratio of the cyclodextrin to the Compound I is from 8:1 to 18:1, preferably from 10:1 to 17:1, more preferably from 11:1 to 16:1, even more preferably from 12:1 to 15:1, still even more preferably from 12.5 to 15:1, and most preferably from 13:1 to 14:1.

According to one embodiment the composition comprises a tonicity agent, preferably the tonicity agent is selected from the group consisting of salts, non-electrolytes, and mixtures thereof, more preferably the tonicity agent is selected from the group consisting of boric acid, inorganic salts, polyols, dextrose, trehalose, urea, and mixtures thereof, even more preferably the tonicity agent is selected from the group consisting of boric acid, sodium chloride, potassium chloride, magnesium chloride, calcium chloride, sodium sulphate, sodium hydrogen phosphate, trisodium citrate, trisodium phosphate, magnesium sulphate, zinc sulphate, sodium carbonate, mannitol, glucose, glycerol, xylitol, sorbitol, propylene glycol, dextrose, trehalose, urea, and combinations thereof, and most preferably the tonicity agent is selected from the group consisting of sodium chloride, potassium chloride, magnesium chloride, mannitol, and mixtures thereof. According to a further embodiment the composition comprises the tonicity agent in an amount such that the composition exhibits an osmolality of from 100 to 500 mOsmol/kg, preferably from 225 to 430 mOsmol/kg, and most preferably from 250 to 350 mOsmol/kg.

According to one embodiment the composition comprises a buffer, preferably the buffer is selected from the group consisting of phosphate buffer, phosphate citrate buffer, citrate buffer, tartrate buffer, borate buffer, malate buffer, succinate buffer, acetate buffer, acetate borate buffer, MES (2-(N-morpholino)ethanesulfonic acid), HEPES (2-(4-(2-hydroxyethy1)I-piperazinyl-ethanesulfonic acid), BES (N,N-bis(2 -hydroxyethyl)-2-aminoethanesulfonic acid), MOPS (3-(N-morpholino)propanesulfonic acid), TRIS (tris(hydroxymethyl)amino-methane), and BIS-TRIS (bis(2-hydroxyethyl)amino-tris(hydroxymethyl)methane, more preferably the buffer is selected from the group consisting of phosphate buffer, phosphate citrate buffer, citrate buffer, borate buffer, or TRIS, and most preferably the buffer is a citrate buffer. According to a further embodiment the pH of the composition is from 5.0 to 8.0, preferably from 5.5 to 7.9, more preferably 6.0 to 7.8, even more preferably from 6.2 to 7.5, and most preferably about 6.5 to 7.4.

According to one embodiment the composition further comprises a preservative, preferably the preservative is selected from the group consisting of cetrimide, benzododecinium chloride, benzalkonium chloride, thiomersal, alexidine, polyquad, hydrogen peroxide, sodium hydroxymethylglycinate, polyhexanide, sorbic acid, sodium perborate, oxyd (stabilized sodium chlorite with hydrogen peroxide), SofZia^{®} (preservative system composed of boric acid, propylene glycol, sorbitol, and zinc chloride), and mixtures thereof, and most preferably the preservative is cetrimide, benzalkonium chloride, or a mixture thereof.

According to one embodiment the composition comprises: 0.01 to 0.5 wt.-%, preferably 0.02 to 0.3 wt.-%, based on the total weight of the composition, of the Compound I, 1 to 30 wt.-%, preferably 2 to 20 wt.-%, based on the total weight of the composition, of the cyclodextrin, preferably 2-hydroxypropyl-β-cyclodextrin, optionally 0.7 to 1.2 wt.-%, based on the total weight of the composition, of a buffer, preferably a phosphate buffer or citrate buffer, optionally 0.1 to 7 wt.-%, preferably 0.2 to 5 wt.-%, based on the total weight of the composition, of a tonicity agent, preferably sodium chloride or mannitol, sodium hydroxide and/or hydrochloric acid for pH adjustment, and q.s. ad 100 wt.-% water.

According to a further embodiment the composition, at a shear rate of 10 s⁻¹, exhibits a viscosity of less than 350 mPa·s at 35°C, preferably less than 250 mPa·s at 35°C, more preferably less than 200 mPa·s at 35°C, even more preferably less 150 mPa·s at 35°C, and most preferably less than 100 mPa·s at 35°C, and/or the composition exhibits an osmolality of from 100 to 500 mOsmol/kg, preferably from 225 to 430 mOsmol/kg, and most preferably from 250 to 350 mOsmol/kg.

In the following, terms that are used in the present invention are explained:
For the purpose of the present invention, the term "buffer-free" means that the composition comprises exclusively ingredients that are no buffers in the meaning the present invention. For the purpose of the present invention, a "buffer" may be a buffer system, e.g. a mixture of an acid and its conjugated base, or a buffer substance. Examples for buffers with the meaning of the present invention are phosphate buffers, phosphate citrate buffers, citrate buffers, tartrate buffers, borate buffers, malate buffers, succinate buffers, acetate buffers, acetate borate buffers, MES (2-(N-morpholino)ethanesulfonic acid), HEPES (2-(4-(2-hydroxyethyl)1-piperazinyl-ethanesulfonic acid), BES (N,N-bis(2-hydroxyethyl)-2-aminoethanesulfonic acid), MOPS (3-(N-morpholino)propanesulfonic acid), TRIS (tris(hydroxymethyl)aminomethane), or BIS-TRIS (bis(2-hydroxyethyl)amino-tris(hydroxymethyl)methane.

In one embodiment, the composition is buffer-free, if the buffer capacity *P*ᵢ of the composition is less than 0.005, and preferably less than 0.0005. To determine the buffer capacity *P*ᵢ, a 0.1 N solution of sodium hydroxide is added to 10 mL of the composition to be tested until a pH change of 0.3 units occurs. The buffer capacity *P*ᵢ can then be determined as follows: ${P}_{i} = \frac{mL alkaline solution ⋅ 0.1}{pH change ⋅ 10}$ (cf. Dolder, Skinner, Ophthalmika, 4. Volume, Wissenschaftliche Verlagsgesellschaft, page 606, chapter 4.1.2.4).

The term "viscosity" is understood herein, unless noted otherwise, as the dynamic viscosity, which is also known as the absolute viscosity. According to the present invention, the shear viscosity was evaluated since this is more representative for every material compared to a simple viscosity value at a specific shear rate. To this end, the shear viscosity was determined by a Kinexus Rheometer (NETZSCH-Gerätebau GmbH, D-95100 Selb/Bayern) using the concentric cylinder geometry, which is ideal for low viscosity fluids. The samples were measured at 35°C, which is representative for the temperature of the surface of the eye, in a shear range from 0.01-10000 s⁻¹. The viscosity refers, unless noted otherwise, to the viscosity of the composition at relevant temperatures and for a range of shear rates.

"TRPV1 receptor" refers to the Transient Receptor Potential Vanilloid 1 that has been characterized through molecular cloning and pharmacology (see e.g., Caterina MJ, et al., Nature 1997; 389:816-824). TRPV1 receptor activity can be measured as described in WO2005/120510.

The language "effective amount" of the compounds described herein, refers to that amount of a therapeutic compound necessary or sufficient to perform its intended function within a mammal. An effective amount of the therapeutic compound can vary according to factors such as the amount of the causative agent already present in the mammal, the age, sex, and weight of the mammal, and the ability of the therapeutic compounds of the present disclosure to treat the ocular surface disorder and/or symptoms thereof in the mammal.

As used herein, the term "treat", "treating" or "treatment" in connection to a disease or disorder refers in some embodiments, to ameliorating the disease or disorder (*i.e*., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g*., stabilization of a discernible symptom), physiologically, (*e.g*., stabilization of a physical parameter), or both. In yet another embodiment, "treat", "treating" or "treatment" refers to preventing or delaying the onset or development or progression of the disease or disorder or symptom thereof.

As used herein, the term "subject" or "patient" refers to human and non-human mammals, including but, not limited to, primates, rabbits, pigs, horses, dogs, cats, sheep, and cows. In particular embodiments, a subject or patient is a human. In some embodiments, the term "patient" or "subject" refers to a human being who is diseased with the condition (*i.e*., disease or disorder) described herein and who would benefit from the treatment. As used herein, a subject is "in need of" a treatment if such subject (patient) would benefit biologically, medically or in quality of life from such treatment. In particular embodiments, the subject is an adult human of at least about 18 years of age. In some embodiments, the subject is an adult human from about 18 years of age to about 75 years of age. In some embodiments, the subject is a child of up to about 18 years of age.

As used herein, "ocular surface" refers to the outer surface of the eye, which anatomically comprises the cornea (with epithelium, bowman layer, stroma, Descement membrane, endothelium), conjunctiva, and the corneo-scleral junction, i.e. limbus.

As used herein, "pain" refers to constant or intermittent sensation of actual pain described as but not limited to stabbing, dull, sharp, or ache. Pain may also refer to similar related descriptors such as but not limited to burning, stinging, grittiness, foreign body sensation, dryness, sandy, tired, itchy, irritated, sensitivity to light.

As used herein, "ocular surface pain" refers to pain on the surface of the eye, e.g., cornea. Ocular pain may be nociceptic pain, which is generally caused by external physical or chemical damaging stimuli such as corneal surgery, inflammation, or other damage to the corneal surface. Ocular pain may also result from neuropathic pain, which may occur due to direct damage to the neurons of the body, resulting in messages of pain being sent to the central nervous system and brain regardless of the presence of noxious stimuli. As used herein "ocular surface pain" includes both nociceptic pain and neuropathic pain.

As used herein, the term "visual analog scale" (VAS) is a measure of pain intensity where a subject typically marks a place on a scale that aligns with their level of pain. The pain is marked in a range of "no pain" (score of 0) and "pain as bad as it could be" or "worst imaginable pain" (score of 100). See e.g., Hawker, et al., Arthritis Care & Research 63(11), pp. S240-S252 (November 2011). There are several other well-designed pain scales that may be used to help assess the extent of pain. The numerical rating scale (NRS) is often used, in which subjects use numbers to rate pain. The number scale may be from 1-10, or 1-100. The Wong-Baker FACES Pain Scale combines pictures and numbers for pain ratings. It can be used in children over the age of 3 and in adults. Six faces depict different expressions, ranging from happy to extremely upset. Each is assigned a numerical rating between 0 (smiling) and 10 (crying). The Verbal Pain Intensity Scale uses wordings on a scale to rate pain intensity: No Pain / Mild Pain / Moderate Pain / Severe Pain Very Severe Pain / Worst Possible Pain.

As used herein, "ocular hyperemia" refers to redness of the ocular surface. Ocular hyperemia may be a clinical marker for inflammation and/or ocular irritation. Ocular hyperemia is typically measured using the McMonnies scale, at values from 0 to 5, based on standard photographs.

As used herein, "placebo" refers to an ophthalmic formulation that includes all the components of the administered drug composition without the drug.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, e.g., means that, unless the context clearly dictates otherwise, the term "obtained" does not mean to indicate that, e.g., an embodiment must be obtained by, e.g., the sequence of steps following the term "obtained" though such a limited understanding is always included by the terms "obtained" or "defined" as a preferred embodiment.

Wherever the terms "including" or "exhibiting" are used, the terms must be considered equivalent to the term "comprising".

According to the present invention, an aqueous ophthalmic composition is provided comprising: 1-(6-fluoro-5-((((1r,3r)-3-(4-fluoro-3(trifluoromethyl)phenoxy)-cyclobutyl)-amino)methyl)isoquinolin-8-yl)ethane-1.2-diol (Compound I), and a cyclodextrin. The molar ratio of the cyclodextrin to the Compound I is from 5:1 to 19:1.

Preferred embodiments of the present invention are described in the following. These embodiments and specifications apply mutatis mutandis to the inventive method and the inventive uses.

### Compound I

The aqueous ophthalmic composition of the present invention comprises 1-(6-fluoro-5-((((1r,3r)-3-(4-fluoro-3(trifluoromethyl)phenoxy)-cyclobutyl)-amino)methylisoquinolin-8-yl)ethane-1.2-diol (Compound I) having the following structure:

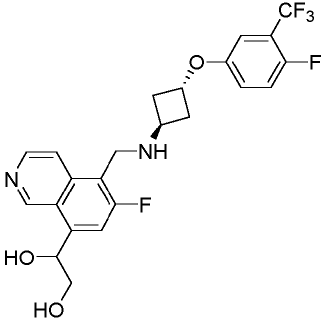

For the purpose of the present invention, the term "Compound I" encompasses the free form of Compound I as well as a salt, a hydrate, a co-crystal, or a polymorph thereof.

Compound I may be amorphous or may exist in one or more different crystalline states (polymorphs), which may have different macroscopic properties such as stability or show different biological properties such as activities. The present invention relates to amorphous and crystalline forms of Compound I, mixtures of different crystalline states of Compound I, as well as amorphous or crystalline salts thereof. As used herein "polymorph" refers to crystalline forms having the same chemical composition but different spatial arrangements of the molecules, atoms, and/or ions forming the crystal.

According to one embodiment, Compound I has the name (S)-1-(6-fluoro-5-((((1r,3S)-3-(4-fluoro-3-(trifluoromethyl)phenoxy)cyclobutyl)amino)methyl)isoquinolin-8-yl)ethane-1,2-diol, and has the structure shown below: 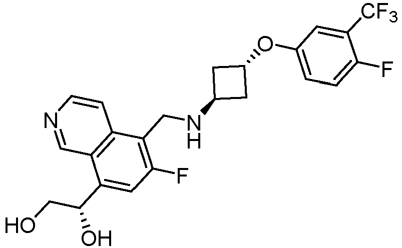 (Crystalline form A).

As described here, Compound I is a stereoisomer that may be present either in its racemic form or in an enantiomeric excess (ee) of the isomer shown above, wherein the isomer shown above is present in at least 90% ee, at least 95% ee, at least 96% ee, at least 97% ee, at least 98% ee, or at least 99% ee.

Crystalline form A may be characterized as such by powder X-ray diffraction (XRPD) wherein the pattern resulting from the analysis comprises significant peaks at characteristic 2-theta (2θ) angles. Crystalline form A may be characterized, for example, by an X ray diffraction pattern having three or more peaks at 2θ values selected from 14.3, 14.8, and 21.8 ± 0.2 °2θ. Crystalline form A may be characterized, for example, by an X-ray diffraction pattern having three or more peaks at 2θ values selected from 12.5, 14.3, 14.8, 21.8, and 22.6 ± 0.2 °2θ. Yet further, crystalline form A may be characterized, for example, by an X-ray diffraction pattern having 3 or more, 4 or more, 5 or more, 6 or more, or 7 or more peaks at 2θ values selected from 12.5, 14.3, 14.8, 20.1, 21.8, 22.6, and 23.2 ± 0.2 °2θ. As described herein, the X-ray diffraction peaks are measured using CuK_{α} radiation having a wavelength of 0.15418 nm. Methods for preparing and characterizing crystalline form A are described in WO 2024/062389 A1.

Any chemical formula given herein is also intended to represent unlabeled forms as well as isotopically labeled forms of the compounds. Isotopically labeled compounds have structures depicted by the formulae given herein except that one or more atoms are replaced by an atom having a selected atomic mass or mass number. Isotopes that can be incorporated into compounds of the disclosure include, for example, isotopes of hydrogen, carbon, nitrogen, and oxygen, such as ²H, ³H, ¹¹C, ¹³C, ¹⁴C, and ¹⁵N. Accordingly, it should be understood that compositions and methods of the present invention can or may involve compounds that incorporate one or more of any of the aforementioned isotopes, including for example, radioactive isotopes, such as ³H and ¹⁴C, or those into which non-radioactive isotopes, such as ²H and ¹³C are present. Such isotopically labelled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. Isotopically-labeled compounds can generally be prepared by conventional techniques known to those skilled in the art, e.g., using an appropriate isotopically-labeled reagents in place of the non-labeled reagent previously employed.

The present invention encompasses embodiments that include all pharmaceutically acceptable salts of Compound I. The phrase "pharmaceutically acceptable" as employed herein refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salt" refers to derivatives of the disclosed compound, wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts of the parent compound formed, for example, from non-toxic inorganic or organic acids. The pharmaceutically acceptable salts can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Suitable pharmaceutically acceptable salts are, e.g., the counterions present in drug products listed in the US FDA Orange Book database. For example, preferred pharmaceutically acceptable salts include, but are not limited to, mineral or organic acid salts of basic residues such as amines. For example, the salt can be a hydrochloride salt.

A "co-crystal" in the meaning of the present invention refers to a crystalline single phase material composed of two or more different molecules and/or ionic compounds in a stoichiometric ratio.

The term "hydrate" in connection with Compound I refers to Compound I, which contains water or its constituent elements (i.e. H and OH). Preferably, a hydrate of Compound I incorporates water molecules in the crystalline structure but does not alter the chemical structure of Compound I. It is to be understood that such hydrates of Compound I also include hydrates of pharmaceutically acceptable salts of the corresponding compounds.

According to one embodiment, the aqueous ophthalmic composition comprises Compound I in an amount from 0.01 to 1 wt.-%, preferably from 0.02 to 0.7 wt.-%, more preferably from 0.04 to 0.5 wt.-%, and most preferably from 0.06 to 0.3 wt.-%, based on the total weight of the composition.

Unless otherwise specified, the weight of Compound I is the weight of the compound itself, not that of a salt, a hydrate, or a co-crystal thereof. For example, the weight of a corresponding salt of Compound I suitable for the methods, compositions, or combinations disclosed herein may be calculated based on the ratio of the molecular weights of the salt and compound itself.

According to one embodiment, Compound I, preferably crystalline form A, is provided in pure or free form. According to another embodiment, Compound I, preferably crystalline form A, is provided in form of a salt, a hydrate, or a co-crystal of Compound I.

### Cyclodextrin

In addition to Compound I, the aqueous ophthalmic composition of the present invention comprises a cyclodextrin.

Cyclodextrins are a family of cyclic oligosaccharides, consisting of a macrocyclic ring of glucose subunits joined by α-1,4 glycosidic bonds, and can be produced from starch by enzymatic conversion. Typically, cyclodextrins contain a number of glucose monomers ranging from six to eight units in a ring, creating a cone shape, wherein α-cyclodextrin contains 6 glucose subunits, a β-cyclodextrin contains 7 glucose subunits, and a γ-cyclodextrin contains 8 glucose subunits.

In an aqueous environment cyclodextrins can form inclusion complexes with lipophilic molecules through a process in which water molecules located inside the central cavity of the cyclodextrin are replaced by either the whole lipophilic molecule or by some lipophilic structure of this molecule. Thereby the physicochemical properties of the complexed molecule such as aqueous solubility and chemical stability may be altered. The water-solubility or other properties of a cyclodextrin can be manipulated by chemical modification of the hydroxyl groups. O-Methylation and acetylation are typical conversions. Reaction with propylene oxide gives hydroxypropylated derivatives, and the primary alcohols can be tosylated. The degree of derivatization is adjustable and may range from partial to full modification of the hydroxyl groups.

For the purpose of the present invention, the term "derivates thereof" in connection with cyclodextrin refers to modified cyclodextrin, wherein the hydroxyl groups of the cyclodextrin are partially or fully replaced by suitable functional groups or substituents, e.g. hydroxyethyl, hydroxypropyl, methyl, carboxymethyl, or sulfobutyl.

The molar substitution (MS) defines the average number of substituents that have reacted with one glucose subunit of the cyclodextrin. The values can range from 0 (no substitution) to 3 when all three hydroxyl groups are substituted.

The inventors of the present invention found that Compound I can effectively form inclusion complexes with cyclodextrin in aqueous compositions, in particular, within pH ranges that are relevant for ophthalmic applications.

According to one embodiment, the cyclodextrin is selected from the group consisting of α-cyclodextrin, β-cyclodextrin, γ-cyclodextrin, derivatives thereof, and mixtures thereof, preferably β-cyclodextrin, derivatives thereof, and mixtures thereof. The cyclodextrin derivates may have a molar substitution (MS) from 0.1 to 3, preferably from 0.2 to 2.5, more preferably from 0.3 to 2, and preferably from 0.4 to 1.5.

According to one embodiment, the cyclodextrin is selected from the group consisting of α-cyclodextrin, β-cyclodextrin, methyl-β-cyclodextrin, 2-hydroxypropyl-β-cyclodextrin, 2-hydroxyethyl-β-cyclodextrin, sulfobutylether-β-cyclodextrin, heptakis (2,6-di-*O*-methyl)-β-cyclodextrin, heptakis (2, 3, 6-tri-*O*-methyl)-β-cyclodextrin, succinyl-β-cyclodextrin, 6-*O*-α-Maltosyl-β-cyclodextrin, 2-hydroxypropyl-γ-cyclodextrin, 2-hydroxyethyl-γ-cyclodextrin, and mixtures thereof. Preferably the cyclodextrin may be selected from the group consisting of β-cyclodextrin, methyl-β-cyclodextrin, 2-hydroxypropyl-β-cyclodextrin, 2-hydroxyethyl-β-cyclodextrin, sulfobutylether-β-cyclodextrin, heptakis (2,6-di-*O*-methyl)-β-cyclodextrin, heptakis (2, 3, 6-tri-*O*-methyl)-β-cyclodextrin, succinyl-β-cyclodextrin, 6-*O*-α-Maltosyl-β-cyclodextrin, and mixtures thereof, and most preferably the cyclodextrin may be 2-hydroxypropyl-β-cyclodextrin.

According to one embodiment, the cyclodextrin is 2-hydroxypropyl-β-cyclodextrin. The 2-hydroxypropyl-β-cyclodextrin may have a molar substitution (MS) from 0.1 to 3, preferably from 0.2 to 2.5, more preferably from 0.3 to 2, and preferably from 0.4 to 1.5. According to one embodiment, the 2-hydroxypropyl-β-cyclodextrin has a molar substitution (MS) from 0.4 to 1.5, preferably from 0.45 to 1.2, more preferably from 0.5 to 1.1, and preferably from 0.55 to 1.

It is known to the skilled person that the molecular weight of a cyclodextrin depends on its number of glucose subunits and substituents. For example, α-cyclodextrin has a molecular weight of about 973 g/mol, β-cyclodextrin has a molecular weight of about 1135 g/mol, and γ-cyclodextrin has a molecular weight of about 1297 g/mol. The molecular weight of derivates thereof may be calculated by adding the molecular weight of all substituents attached to the cyclodextrin molecule.

According to one embodiment, the cyclodextrin has a molecular weight from 973 g/mol to 1700 g/mol, preferably from 1000 g/mol to 1600 g/mol, more preferably from 1100 g/mol to 1500 g/mol, and most preferably from 1200 g/mol to 1450 g/mol.

It was found by the inventors that in an aqueous environment the enclosed Compound I molecule may dissociate from the cyclodextrin molecules through complex dilution and the free Compound I molecule and the cyclodextrin molecule may be in a dynamic equilibrium with the Compound I-cyclodextrin-complex. In the aqueous tear fluid cyclodextrins may act as carriers by keeping the Compound I molecules in solution and delivering them, for example, to the cornea of the eye, where they partition from the cyclodextrin cavity, for example, into the lipophilic membrane of the cornea. The large hydrophilic cyclodextrin molecules and Compound I-cyclodextrin-complexes remain in the aqueous skin exterior, e.g. the aqueous tear fluid.

According to the present invention, the molar ratio of the cyclodextrin to the compound I is from 5:1 to 19:1.

The inventors of the present invention surprisingly found that at this specific molar ratio range of the cyclodextrin to the Compound I the delivery of Compound I through the corneal tissue was surprisingly high. This provides the possibility to efficiently administer Compound I to the ocular surface in form of an aqueous solution. As noted above, aqueous solutions are preferred for ophthalmic application because they are better tolerated by patients, show better patient compliance, better shelf-life stability, and are easier to manufacture.

According to one embodiment, the molar ratio of the cyclodextrin to the Compound I is from 8:1 to 18:1, preferably from 10:1 to 17:1, more preferably from 11:1 to 16:1, even more preferably from 12:1 to 15:1, still even more preferably from 12.5 to 15:1, and most preferably from 13:1 to 14:1.

According to one embodiment, the mass concentration ratio of the Compound I is from 20:1 to 60:1, preferably from 25:1 to 55:1, more preferably from 30:1 to 50:1, even more preferably from 30:1 to 45:1, and most preferably from 40:1 to 43:1.

### The aqueous ophthalmic composition

According to the present invention, the aqueous ophthalmic composition comprises Compound I, and a cyclodextrin, wherein the molar ratio of the cyclodextrin to the Compound I is from 5:1 to 19:1.

For the purpose of the present invention, the expression "aqueous ophthalmic composition" refers to composition consisting of a single liquid aqueous phase, wherein the composition is suitable for ophthalmic application. The liquid aqueous phase comprises water, and preferably water for injection purposes. In other words, the aqueous ophthalmic composition is an aqueous ophthalmic solution, wherein the Compound I and the cyclodextrin are present in a dissolved state. The same is true for additional components of the aqueous ophthalmic composition, if present. According to one embodiment, the aqueous ophthalmic composition is not in form of a suspensions and/or emulsion.

According to one embodiment, an aqueous ophthalmic composition is provided, wherein the aqueous ophthalmic composition is in form a liquid phase, which comprises Compound I, a cyclodextrin, and water, wherein the molar ratio of the cyclodextrin to the Compound I is from 5:1 to 19:1. According to a further embodiment an aqueous ophthalmic solution is provided, wherein the aqueous ophthalmic composition comprises Compound I, a cyclodextrin, and water, wherein the molar ratio of the cyclodextrin to the Compound I is from 5:1 to 19:1.

According to one embodiment the composition comprises Compound I in an amount from 0.01 to 1 wt.-%, preferably from 0.02 to 0.7 wt.-%, more preferably from 0.04 to 0.5 wt.-%, and most preferably from 0.06 to 0.3 wt.-%, based on the total weight of the composition. According to another embodiment the composition comprises Compound I in an amount from 0.01 to 1 % w/v (weight/volume), preferably from 0.02 to 0.7 % w/v, more preferably from 0.04 to 0.5 % w/v, and most preferably from 0.06 to 0.3 % w/v, based on the total volume of the composition. According to a further embodiment, the composition comprises Compound I in an amount from 0.1 to 10 mg/ml, preferably from 0.2 to 7 mg/ml, more preferably from 0.4 to 5 mg/ml, and most preferably from 0.6 to 3 mg/ml.

In addition or alternatively, the composition comprises the cyclodextrin in an amount from 0.01 to 50 wt.-%, preferably from 0.05 to 40 wt.-%, more preferably from 0.07 to 30 wt.-%, and most preferably from 0.1 to 20 wt.-%, based on the total weight of the composition. According to another embodiment, the composition comprises the cyclodextrin in an amount from 0.01 to 50 % w/v (weight/volume), preferably from 0.05 to 40 % w/v, more preferably from 0.07 to 30 % w/v, and most preferably from 0.1 to 20 % w/v, based on the total volume of the composition. According to a further embodiment, the composition comprises the cyclodextrin in an amount from 0.1 to 500 mg/ml, preferably from 0.5 to 400 mg/ml, more preferably from 0.7 to 300 mg/ml, and most preferably from 1 to 200 mg/ml.

In addition to the above-described components, the aqueous ophthalmic composition of the present invention can comprise further components.

In one embodiment, the inventive composition comprises an agent to adjust the isotonicity, i.e. a tonicity agent. Non-electrolytes and/or electrolytes are suitable tonicity agents. Examples of suitable non-electrolytes to adjust the isotonicity are dextrose, glycerin, propylene glycol, sorbitol, mannitol, glucose, urea, polyethylene glycol and mixtures thereof. Examples of suitable electrolytes to adjust the isotonicity are boric acid, magnesium sulphate, magnesium chloride, zinc sulphate, sodium chloride, potassium chloride, calcium chloride, sodium sulfate, sodium hydrogen phosphate, trisodium citrate, trisodium phosphate, and mixtures thereof.

According to one embodiment the tonicity agent is selected from the group consisting of salts, non-electrolytes, and mixtures thereof. The salts may be inorganic salts or organic salts. According to one embodiment, the tonicity agent is selected from the group consisting of boric acid, inorganic salts, polyols, dextrose, trehalose, urea, and mixtures thereof, preferably the tonicity agent is selected from the group consisting of boric acid, sodium chloride, potassium chloride, magnesium chloride, calcium chloride, sodium sulphate, sodium hydrogen phosphate, trisodium citrate, trisodium phosphate, magnesium sulphate, zinc sulphate, mannitol, glucose, glycerol, xylitol, sorbitol, propylene glycol, dextrose, trehalose, urea, and combinations thereof, and most preferably the tonicity agent is selected from the group consisting of sodium chloride, potassium chloride, magnesium chloride, mannitol, and mixtures thereof.

The composition may comprise the tonicity agent to adjust the isotonicity in an amount that is sufficient to provide the composition isotonically to natural tear fluid. According to one embodiment, the composition comprises the tonicity agent in an amount such that the composition exhibits an osmolality of from 100 to 500 mOsmol/kg, preferably from 225 to 430 mOsmol/kg, and most preferably from 250 to 350 mOsmol/kg. For example, the composition may comprise the tonicity agent in an amount from 0.05 to 10 wt.-%, preferably 0.1 to 8 wt.-%, more preferably from 0.15 to 7 wt.-%, and most preferably from 0.2 to 5 wt.-%, based on the total weight of the composition. According to one embodiment the composition comprises the tonicity agent in an amount from 0.05 to 10 % w/v, preferably 0.1 to 8 % w/v, more preferably from 0.15 to 7 % w/v, and most preferably from 0.2 to 5 % w/v, based on the total volume of the composition.

According to one embodiment, the tonicity agent is a salt, preferably the salt is selected from the group consisting of sodium chloride, potassium chloride, magnesium chloride, calcium chloride, sodium sulphate, sodium hydrogen phosphate, trisodium citrate, trisodium phosphate, magnesium sulphate, zinc sulphate, and mixtures thereof, more preferably the salt is selected from the group consisting of sodium chloride, potassium chloride, magnesium chloride, calcium chloride, sodium sulphate, trisodium citrate, magnesium sulphate, zinc sulphate, and mixtures thereof, and most preferably the salt is selected from the group consisting of sodium chloride, potassium chloride, magnesium chloride, and mixtures thereof. The composition may comprise the salt in an amount from 0.01 to 0.5 wt.-%, preferably from 0.02 to 0.4 wt.-%, more preferably from 0.03 to 0.3 wt.-%, even more preferably from 0.04 to 0.4 wt.-%, and most preferably from 0.05 to 0.1 wt.-%, based on the total weight of the composition. According to one embodiment, the composition comprises the salt in an amount from 0.01 to 0.5 % w/v, preferably from 0.02 to 0.4 % w/v, more preferably from 0.03 to 0.3 % w/v, even more preferably from 0.04 to 0.4 % w/v, and most preferably from 0.05 to 0.1 % w/v, based on the total volume of the composition.

According to one embodiment, the aqueous ophthalmic composition of the present invention does not comprise a tonicity agent.

The skilled person will appreciate that the pH value of the inventive composition will be in a physiologically tolerable range. According to one embodiment, the pH of the composition is from 5.0 to 8.0, preferably from 5.5 to 7.9, more preferably 6.0 to 7.8, even more preferably from 6.2 to 7.5, and most preferably about 6.5 to 7.4.

The pH value of the inventive composition can be adjusted with acids and/or bases. For example, the pH value may be adjusted with hydrochloric acid and/or sodium hydroxide solution. The hydrochloric acid and/or sodium hydroxide solution may have a concentration of 1 mol/I (1 N). In one embodiment, the inventive composition comprises an acid and/or a base in an amount of from 0.01 to 2 wt.%, based on the total weight of the composition, preferably from 0.015 to 1 wt.-%, more preferably from 0.02 to 0.5 wt.-%, and most preferably from 0.02 to 0.05 wt.-%. In one embodiment, the inventive composition comprises an acid and/or a base in an amount of from 0.01 to 2 % w/v, based on the total volume of the composition, preferably from 0.015 to 1 % w/v, more preferably from 0.02 to 0.5 % w/v, and most preferably from 0.02 to 0.05 % w/v.

In addition or alternatively, a buffer may be used to adjust and/or maintain the pH value of the invention composition.

According to one embodiment, the composition of the present invention comprises a buffer, preferably the buffer is selected from the group consisting of phosphate buffer, phosphate citrate buffer, citrate buffer, tartrate buffer, borate buffer, malate buffer, succinate buffer, acetate buffer, acetate borate buffer, MES (2-(N-morpholino)ethanesulfonic acid), HEPES (2-(4-(2-hydroxyethy1)I-piperazinyl-ethanesulfonic acid), BES (N,N-bis(2 -hydroxyethyl)-2-aminoethanesulfonic acid), MOPS (3-(N-morpholino)propanesulfonic acid), TRIS (tris(hydroxymethyl)amino-methane), and BIS-TRIS (bis(2-hydroxyethyl)amino-tris(hydroxymethyl)methane, more preferably the buffer is selected from the group consisting of phosphate buffer, phosphate citrate buffer, citrate buffer, borate buffer, or TRIS, and most preferably the buffer is a citrate buffer.

According to one embodiment, the composition comprises the buffer in an amount such that the composition exhibits a pH from 5.0 to 8.0, preferably from 5.5 to 7.9, more preferably 6.0 to 7.8, even more preferably from 6.2 to 7.5, and most preferably about 6.5 to 7.4. For example, the composition may comprise the buffer in an amount from 0.05 to 2.5 wt.-%, preferably 0.1 to 2 wt.-%, more preferably from 0.5 to 1.5 wt.-%, and most preferably from 0.7 to 1.2 wt.-%, based on the total weight of the composition. According to one embodiment, the composition comprises the buffer in an amount from 0.05 to 2.5 % w/v, preferably 0.1 to 2 % w/v, more preferably from 0.5 to 1.5 % w/v, and most preferably from 0.7 to 1.2 % w/v, based on the total volume of the composition.

According to one embodiment, the buffer has a buffer strength from 1 to 100 mM, preferably from 2 to 80 mM, and most preferably from 5 to 50 mM, and composition comprises the buffer in an amount from 0.05 to 2.5 wt.-%, preferably 0.1 to 2 wt.-%, more preferably from 0.5 to 1.5 wt.-%, and most preferably from 0.7 to 1.2 wt.-%, based on the total weight of the composition.

According to one embodiment, the aqueous ophthalmic composition is buffer-free and/or free of phosphate salts.

According to one embodiment, the inventive composition further comprises a preservative, preferably the preservative is selected from the group consisting of cetrimide, benzododecinium chloride, benzalkonium chloride, thiomersal, alexidine, polyquad, hydrogen peroxide, sodium hydroxymethylglycinate, polyhexanide, chlorhexidine, sorbic acid, sodium perborate, oxyd (stabilized sodium chlorite with hydrogen peroxide), SofZia^{®} (preservative system composed of boric acid, propylene glycol, sorbitol, and zinc chloride), and mixtures thereof, and most preferably the preservative is cetrimide, benzalkonium chloride, or a mixture thereof.

In one embodiment, the inventive composition comprises the preservative in an amount of from 0.001 to 0.05 wt.-%, based on the total amount of the composition, preferably from 0.005 to 0.02 wt.-%. For example, the inventive composition may comprise the preservative in an amount of from 0.001 to 0.05 % w/v, based on the total volume of the composition, preferably from 0.005 to 0.02 % w/v.

In a particularly preferred embodiment, the aqueous ophthalmic composition is free of preservatives.

According to one embodiment the aqueous ophthalmic composition comprises, preferably consists of:
0.01 to 0.5 wt.-%, preferably 0.02 to 0.3 wt.-%, based on the total weight of the composition, of the Compound I,
1 to 30 wt.-%, preferably 2 to 20 wt.-%, based on the total weight of the composition, of the cyclodextrin, preferably 2-hydroxypropyl-β-cyclodextrin,
sodium hydroxide and/or hydrochloric acid for pH adjustment, and
q.s. ad 100 wt.-% water.

The cyclodextrin, preferably 2-hydroxypropyl-β-cyclodextrin, may have a MS from 0.4 to 1.5, preferably from 0.45 to 1.2, more preferably from 0.5 to 1.1, and preferably from 0.55 to 1. According to one embodiment, the 2-hydroxypropyl-β-cyclodextrin has a MS from 0.6 to 0.95, preferably from 0.65 to 0.9, more preferably from 0.7 to 0.8, and most preferably about 0.74.

According to another embodiment the aqueous ophthalmic composition comprises, preferably consists of:
0.01 to 0.5 wt.-%, preferably 0.02 to 0.3 wt.-%, based on the total weight of the composition, of the Compound I,
1 to 30 wt.-%, preferably 2 to 20 wt.-%, based on the total weight of the composition, of the cyclodextrin, preferably 2-hydroxypropyl-β-cyclodextrin,
0.1 to 1.2 wt.-%, based on the total weight of the composition, of a buffer, preferably a phosphate buffer or citrate buffer,
0.15 to 7 wt.-%, preferably 0.2 to 5 wt.-%, based on the total weight of the composition, of a tonicity agent, preferably sodium chloride or mannitol,
sodium hydroxide and/or hydrochloric acid for pH adjustment, and
q.s. ad 100 wt.-% water.

The cyclodextrin, preferably 2-hydroxypropyl-β-cyclodextrin, may have a MS from 0.4 to 1.5, preferably from 0.45 to 1.2, more preferably from 0.5 to 1.1, and preferably from 0.55 to 1. According to one embodiment, the 2-hydroxypropyl-β-cyclodextrin has a MS from 0.6 to 0.95, preferably from 0.65 to 0.9, more preferably from 0.7 to 0.8, and most preferably about 0.74.

The expression "q.s. ad 100 wt.-% water" means, for the purpose of the present invention, that water is added in such an amount, to reach a total amount of 100 wt.-%.

According to one embodiment the aqueous ophthalmic composition comprises, preferably consists of:
0.01 to 0.5 % w/v, preferably 0.02 to 0.3 % w/v, based on the total weight of the composition, of the Compound I,
1 to 30 % w/v, preferably 2 to 20 % w/v, based on the total weight of the composition, of the cyclodextrin, preferably 2-hydroxypropyl-β-cyclodextrin,
sodium hydroxide and/or hydrochloric acid for pH adjustment, and
q.s. ad 100 % w/v water.

The cyclodextrin, preferably 2-hydroxypropyl-β-cyclodextrin, may have a MS from 0.4 to 1.5, preferably from 0.45 to 1.2, more preferably from 0.5 to 1.1, and preferably from 0.55 to 1. According to one embodiment, the 2-hydroxypropyl-β-cyclodextrin has a MS from 0.6 to 0.95, preferably from 0.65 to 0.9, more preferably from 0.7 to 0.8, and most preferably about 0.74.

According to another embodiment the aqueous ophthalmic composition comprises, preferably consists of:
0.01 to 0.5 % w/v, preferably 0.02 to 0.3 % w/v, based on the total weight of the composition, of the Compound I,
1 to 30 % w/v, preferably 2 to 20 % w/v, based on the total weight of the composition, of the cyclodextrin, preferably 2-hydroxypropyl-β-cyclodextrin,
0.1 to 1.2 % w/v, based on the total weight of the composition, of a buffer, preferably a phosphate buffer or citrate buffer,
0.15 to 7 % w/v, preferably 0.2 to 5 % w/v, based on the total weight of the composition, of a tonicity agent, preferably sodium chloride or mannitol,
sodium hydroxide and/or hydrochloric acid for pH adjustment, and
q.s. ad 100 % w/v water.

The cyclodextrin, preferably 2-hydroxypropyl-β-cyclodextrin, may have a MS from 0.4 to 1.5, preferably from 0.45 to 1.2, more preferably from 0.5 to 1.1, and preferably from 0.55 to 1. According to one embodiment, the 2-hydroxypropyl-β-cyclodextrin has a MS from 0.6 to 0.95, preferably from 0.65 to 0.9, more preferably from 0.7 to 0.8, and most preferably about 0.74.

The expression "q.s. ad 100 % w/v water" means, for the purpose of the present invention, that water is added in such an amount, to reach a total volume of 100 % w/v.

In a preferred embodiment, the inventive composition does not contain further substances besides the ones mentioned above.

According to an exemplary embodiment, the aqueous ophthalmic composition comprises, preferably consists of:
1 to 2 mg/ml, preferably about 1.5 mg/ml of the Compound I,
55 to 70 mg/ml, preferably 60 to 65 mg/ml, of 2-hydroxypropyl-β-cyclodextrin, preferably having a MS from 0.55 to 1, more preferably a MS from 0.7 to 0.8, and most preferably a MS of about 0.74,
7 to 8 mg/ml, preferably about 7.8 mg/ml, of a phosphate buffer, preferably sodium dihydrogen phosphate dihydrate (sodium phosphate monobasic, dihydrate),
15 to 20 mg/ml, preferably 19 mg/ml, of mannitol,
sodium hydroxide and/or hydrochloric acid for pH adjustment, and
q.s. ad 1000 ml water.

According to another exemplary embodiment, the aqueous ophthalmic composition comprises, preferably consists of:
0.2 to 3 mg/ml of the Compound I,
8 to 130 mg/ml, preferably 8.7 to 129.9 mg/ml, of 2-hydroxypropyl-β-cyclodextrin, preferably having a MS from 0.55 to 1, more preferably a MS from 0.7 to 0.8, and most preferably a MS of about 0.74,
7 to 8 mg/ml, preferably about 7.8 mg/ml, of a phosphate buffer, preferably sodium dihydrogen phosphate dihydrate (sodium phosphate monobasic, dihydrate),
1 to 5.5 mg/ml, preferably 1.3 to 5 mg/ml, of sodium chloride,
sodium hydroxide and/or hydrochloric acid for pH adjustment, and
q.s. ad 1000 ml water.

### Properties, preparation and use

The skilled person will adapt the physical and chemical properties of the inventive aqueous ophthalmic composition according to the envisaged application.

According to one embodiment, at a shear rate of 10 s⁻¹ the composition exhibits a viscosity of less than 350 mPa·s at 35°C, preferably less than 250 mPa·s at 35°C, more preferably less than 200 mPa·s at 35°C, even more preferably less 150 mPa·s at 35°C, and most preferably less than 100 mPa·s at 35°C. The shear viscosity may be determined by a Kinexus Rheometer (NETZSCH-Gerätebau GmbH, D-95100 Selb/Bayern) equipped with the concentric cylinder geometry at 35°C and for a shear range from 0.01-10000 s⁻¹, preferably for a shear rate of 10 s⁻¹.

Additionally or alternatively, the composition may exhibit an osmolality of from 100 to 500 mOsmol/kg, preferably from 225 to 430 mOsmol/kg, and most preferably from 250 to 350 mOsmol/kg.

The inventive composition may be any suitable form for ophthalmic application. According to one embodiment the composition is in form of an eye lid spray, an eye bath, an eye wash solution, or eye drops.

The inventive composition can be prepared aseptically without difficulties and is well tolerated. According to a further aspect a method for preparing an aqueous ophthalmic composition according to the invention is provided, wherein the method comprises the steps of:
mixing the compound I, the cyclodextrin, and water, and
adjusting the pH to a range from 5.0 to 8.0.

If a buffer and/or a tonicity agent is present, the method may comprise the steps of:
mixing the compound I, the cyclodextrin, the buffer and/or the tonicity agent, and water, and
adjusting the pH to a range from 5.0 to 8.0.

Alternatively, the buffer and/or tonicity agent may be added before, during, and/or after said mixing step.

For example, in a first step the cyclodextrin may be dissolved in an aqueous solution, preferably under aseptic conditions. If necessary, the pH value of the aqueous solution may be adjusted before adding the cyclodextrin. In a second step, the Compound I is added and mixed with said aqueous solution comprising the cyclodextrin, preferably under aspect conditions. Preferably, it is stirred until complete homogenization. If necessary, the pH value of the obtained composition may be adjusted. Subsequently, additional compounds, e.g. a tonicity agent and/or preservative may be added. The final bulk solution is, preferably sterilized by sterile filtration (e.g. at pore size of 0.22 µm). Afterwards, the obtained, preferably sterile, aqueous ophthalmic composition can be packaged in a standard manner.

For the application, the inventive composition may be filled in suitable container, that are preferably designed such that the content may be applied to the eye.

According to a further aspect a container comprising the aqueous ophthalmic composition according to present invention is provided, wherein the container is a single-dose container, preferably a single-dose ophtiole, or a multi-dose container, preferably an ophtiole, or an application system, preferably a tip-seal application system, a pump-based application system, a standard bottle/dropper system, or a multi-dose application system.

In one embodiment of the present invention, the inventive composition is provided in a type of a single-dose container. Single-dose containers are known in the prior art, for example, under the designation single-dose Ophtiole^{®}, and may preferably be made of polyethylene such as LDPE (low density polyethylene). In a particularly preferred embodiment, the inventive composition is provided in a single-dose container free from preservatives.

Alternatively, the container which comprises the inventive composition, may also be a multi-dose container. For example, the container may be a dropping bottle. Suitable dropping bottles are known in the prior art, e.g. under the designation Ophtiole^{®}, and may be made from polyethylene, preferably HDPE (high density polyethylene) or LDPE (low density polyethylene).

The multi-dose container may also be an application system such as a pump-based application system or a tip-seal system. Examples for pump-based application systems are eye sprays or eye dropping pumps such as the COMOD^{®} system or the 3K^{®} system, both produced by the company Ursatec Verpackung GmbH. Examples for the tip-seal system are the Ophthalmic Squeeze Dispenser (OSD^{®}) system from the company AptarGroup, Inc., or the Novelia^{®} system from the company Nemera.

According to one embodiment a dosage spray bottle comprising a composition of the present invention is provided, wherein preferably the composition is in form of an eye lid spray. Thereby, the eye lid spray can be applied to the eye in form of a spray mist. Typically, eye lid sprays are sprayed onto the closed eye lid several times a day. However, it is also possible to apply the inventive eye lid spray from the front or from the side to the open eye.

According to a further aspect, an aqueous ophthalmic composition according to the present invention for use as a medicament is provided. The inventive composition is particularly suitable for treating a disease or a condition of the eye or the organ or tissue surrounding the eye or being connected therewith.

Without being bound by theory, it is hypothesized that Compound I may block the Transient Receptor Potential Vanilloid 1 (TRPV1) receptor. Thus, the present invention may provide a method of treating a disease or a condition of the eye or the organ or tissue surrounding the eye or being connected therewith, preferably for treating ocular surface disorders in a subject, wherein said method includes administering to the subject an effective amount of the inventive composition.

According to one aspect, an aqueous ophthalmic composition according to the present invention is provided for use in treating a disease or a condition of the eye or the organ or tissue surrounding the eye or being connected therewith, preferably for use in treating ocular surface disorders.

Non-limiting examples of ocular surface disorders include ocular surface pain (e.g. chronic ocular surface pain (COSP) or acute ocular surface pain), dry eye disease, Sjogren's Syndrome, conjunctivitis (including keratoconjuctivitis, vernal keratoconjunctivitis, allergic conjunctivitis), Map-Dot-Fingerprint Dystrophy, acanthamoeba, fibromyalgia, Meibomian gland dysfunction, thyroid eye disease, rosacea, ptosis, keratoconus, ocular pain syndrome, Steven-Johnson's syndrome, corneal epitheliopathies, corneal neuropathies (including LASIK induced corneal neuropathies), corneal dystrophies (including recurrent corneal dystrophies), epithelial basement membrane dystrophy, corneal erosions or abrasions (including recurrent corneal erosions or abrasions), ocular surface diseases, blepharitis, graft vs host disease, meibomitis, glaucoma, conjunctivochalasis, keratopathis (including herpetic keratopathy, filamentary keratopathy, band or bullous keratopathy, exposure keratopathy), keratitis (including herpes simplex virus keratitis), iritis, episclentis, corneal surgery, multiple sclerosis, trichiasis, pterygium, neuralgia, xerophthalmia, or patients recovering from neurotrophic keratitis.

According to one embodiment the inventive aqueous ophthalmic composition is provided for use in treating ocular surface pain, dry eye disease, Sjogren's Syndrome, conjunctivitis, Map-Dot-Fingerprint Dystrophy, acanthamoeba, fibromyalgia, Meibomian gland dysfunction, thyroid eye disease, rosacea, ptosis, keratoconus, ocular pain syndrome, Steven-Johnson's syndrome, corneal epitheliopathies, corneal neuropathies, corneal dystrophies, epithelial basement membrane dystrophy, corneal erosions or abrasions, ocular surface diseases, blepharitis, graft vs. host disease, meibomitis, glaucoma, conjunctivochalasis, keratopathis, keratitis, iritis, episclentis, corneal surgery, multiple sclerosis, trichiasis, pterygium, neuralgia, xerophthalmia, or patients recovering from neurotrophic keratitis, and most preferably for use in treating ocular surface pain.

The ocular surface pain may encompass episodic ocular surface pain, acute ocular surface pain, and/or chronic ocular surface pain. Chronic ocular surface pain may last for at least one month, at least two months, at least three months, at least four months, or at least five months.

The ocular surface pain may be associated with one or more of the other ocular surface disorders mentioned above. In some embodiments, the subject suffers from conjunctivitis, subconjunctival hemorrhage, subconjunctival scarring, conjunctival membranes, conjunctival ulceration, superficial punctate epithelial erosions, epithelial defects, lid margin ulceration, lid margin keratinization, symblepharon, ankyloblepharon, trichiasis, anterior blepharitis, punctal auto-occlusion, meibomian gland disease, corneal opacification, dry eye, districhiasis, limbal stem cell failure, or corneal vascularization.

According to one embodiment the ocular surface pain is associated with dry eye disease or Sjogren's Syndrome. The administration of the inventive composition may result in a decrease in the symptoms of dry eye disease. Dry eye disease is generally understood to be a complex, multifactorial condition characterized by inflammation of the ocular surface and lacrimal glands and reductions in the quality and/or quantity of tears. It is believed that up to 30 % of dry eye disease patients suffer from ocular surface pain that may be chronic. Thus, in some embodiments, the invention results in a decrease of at least about 10%, at least about 15%, at least about 20%, or at least about 30% in the symptoms of dry eye disease, including one or more of ocular dryness, ocular discomfort, ocular hyperemia, ocular burning or stinging, grittiness or foreign body sensation, or photophobia.

In some embodiments, the administration of the inventive composition results in a reduction in the subject's ocular pain, compared to a placebo. In some embodiments, the reduction in the subjects ocular pain is at least about 3 when measured on the VAS score, compared to a placebo. In some embodiments, the administration results in a reduction in the subject's ocular pain of at least about 4, at least about 5, at least about 6, at least about 7, at least about 8, at least about 9, or at least about 10, when measured on the VAS score, compared to a placebo. In some embodiments, the administration results in a reduction in the subject's pain of at least about 10%, at least about 15%, at least about 20%, or at least about 25%, compared to a placebo. In some embodiments, the administration of the compound of formula I results in a reduction in the subject's pain of at least about 2 compared to a placebo, as measured by the VAS score, about half hour after the administration, about one hour, about 2 hours, or about 2-4 hours after the administration.

In some embodiments, the administration of the inventive composition results in reduced ocular hyperemia (redness of the eye), compared to placebo. In particular embodiments, the administration of the compound of formula I results in reduced grade 1, grade 2, grade 3, or grade 4 hyperemia compared to placebo. In some embodiments, the administration results in a reduction in ocular hyperemia score of at least about 1, at least about 2, at least about 3, at least about 4, or at least about 5, on the McMonnies scale. In some embodiments, the ocular hyperemia is associated with one or more of the ocular surface disorders mentioned above.

According to one embodiment, the composition is administered to the ocular surface of the subject, e.g., any part of the cornea, conjunctiva, or to the cul de sac of the eye. The inventive composition may be administered to the subject one to six times a day, e.g., one, two, three, or four times a day. In some embodiments, the composition is administered to the subject for a period of at least about one month, at least about two months, or at least about three months. In some embodiments, the composition is administered to the subject for a period of at least about 12 weeks.

Scope and subject-matter of the present invention will be illustrated by the following examples based on selected embodiments, that shall not be understood as limitations or restrictions.

### Examples

### 1. Materials

| | |
|---|---|
| Compound I: | (S)-1-(6-fluoro-5-((((1r,3S)-3-(4-fluoro-3(trifluoromethyl)phenoxy)-cyclobutyl)-amino)methyl)isoquinolin-8-yl)ethane-1.2-diol (crystalline form A) |
| HPbCD: | 2-Hydroxypropyl-β-cyclodextrin, molecular substitution (MS): 0.74, molecular weight: 1435.44 g/mol. |
| | (The molecular weight was calculated by the following equation: |
| | $MW \left(\frac{g}{mol}\right) = 1135 + 58 n$ |
| | where 1135 = molecular weight of the base structure of β-cyclodextrin, |
| | n = degree of substitution (DS) ranging from 2.8-10.5. |
| | The degree of substitution was calculated by multiplying the MS value with 7, which corresponds to a DS value of 5.18). |
| Phosphate buffer: | Sodium phosphate monobasic, dihydrate; buffer strength: 50 mM |
| HCl: | Hydrochloric acid, 1 N solution |
| NaOH: | Sodium hydroxide, 1 N solution |

### 2. Example 1 - Preparation of aqueous ophthalmic compositions

Compounding of the compositions was performed in a clean room zone of at least grade C. If not stated otherwise, all manufacturing steps are performed at room temperature (15 - 25°C) under GMP conditions. For preparing the composition, a stainless steel vessel was used (C45, HCDSMX 20, Dipl.-Ing. Wilhelm Schmidt GmbH, 64342 Seeheim-Jugenheim, Germany), which was equipped with an agitator.

8000 g water for injection was added to the stainless steel vessel (agitator speed: 600 rpm ± 50 rpm). The mixture was cooled to a nominal temperature of 25°C, while the agitator speed of 600 rpm ± 50 rpm was kept throughout the whole compounding process.

Subsequently, 78 g buffer solution was added progressively into the compounding vessel, and mixed for at least 5 minutes until solution was homogeneous in appearance. If necessary, the pH was adjusted to the target value of 6.0 with HCl and/or NaOH.

As a next step, HPbCD, Compound I, and mannitol were added subsequently to the vessel in the amounts disclosed in Table 1 below. First of all, HPbCD was added carefully and lowly to avoid lump formation and material loss, followed by mixing at least 5 minutes until it was completely dissolved. Afterwards, Compound I was slowly added and mixed thoroughly for at least 2 hours. It was ensured visually that all of the material was added to the mixture. After complete dissolution the pH was adjusted to a target value of 6.5 with HCl and/or NaOH solution. Subsequently, mannitol was slowly added, and the composition was mixed at least 5 minutes until it was completely dissolved. Finally, water for injection was added to a final quantity of 10 L.

The obtained compositions were filtered through a 0.22 µm filter to reduce bioburden (Millipore Durapore^{®} 0.22 µm, hydrophilic CVGL75S01), and subsequently through a sterilized 0.22 µm filter (Millipore Durapore^{®} 0.22 µm, hydrophilic CVGL75S01).

The ingredients and properties of the prepared aqueous ophthalmic compositions are listed in Table 1 below.

**Table 1: Composition and properties of aqueous ophthalmic compositions of Example 1 (comp.: comparative).**

| **Sample** | **1** | **2** | **3 (comp.)** | **4 (comp.)** | **5 (comp.)** | **6 (comp.)** |
|---|---|---|---|---|---|---|
| Compound I | 1.5 mg/ml | 1.5 mg/ml | 1.5 mg/ml | 0.6 mg/ml | 0.2 mg/ml | 0.2 mg/ml |
| HPbCD | 60 mg/ml | 65 mg/ml | 100 mg/ml | 65 mg/ml | 65 mg/ml | 100 mg/ml |
| Mannitol | 19 mg/ml | 19 mg/ml | 19 mg/ml | 19 mg/ml | 19 mg/ml | 19 mg/ml |
| Phosphate buffer (50 mM) | 7.8 mg/ml | 7.8 mg/ml | 7.8 mg/ml | 7.8 mg/ml | 7.8 mg/ml | 7.8 mg/ml |
| HCl | q.s. to pH 6.5 | q.s. to pH 6.5 | q.s. to pH 6.5 | q.s. to pH 6.5 | q.s. to pH 6.5 | q.s. to pH 6.5 |
| NaOH | q.s. to pH 6.5 | q.s. to pH 6.5 | q.s. to pH 6.5 | q.s. to pH 6.5 | q.s. to pH 6.5 | q.s. to pH 6.5 |
| Water for injection | q.s. to 1000 ml | q.s. to 1000 ml | q.s. to 1000 ml | q.s. to 1000 ml | q.s. to 1000 ml | q.s. to 1000 ml |
| HPbCD:Compound I concentration ratio | 40 | 43.3 | 66.67 | 108.3 | 325 | 500 |
| HPbCD:Compound I molar ratio | 13:1 | 14:1 | 22:1 | 35:1 | 106:1 | 163:1 |
| pH | 6.44 | 6.4 | 6.48 | 6.43 | 6.46 | 6.6 |
| Osmolality [mOsmol/kg] | 281 | 287 | 320 | 290 | 290 | 344 |

### 3. Example 2 - Permeability study

In this study, the permeability of Samples 1 to 6 listed in Table 1 above, was tested on a epicorneal tissue model (MatTek EpiCorneal tissues).

### 3.1. Materials

Verapamil hydrochloride was procured by Pharmaron (Exton) Lab Services LLC from Sigma (St. Louis, MO,USA) and used as the internal standard (IS) for the unidirectional flux experiment. Phosphate-buffered saline (PBS, Cat. 10010 from ThermoFisher Scientific, Waltham, MA, USA) was adjusted to pH 7.4, 1% w/v bovine serum albumin (BSA, Cat. 5217 from Tocris Bioscience, Bristol, UK) was added and used as the receiver buffer.

MatTek EpiCorneal tissue-free inserts with surface area of 0.6 cm² (Cat. MILCEL-MTK-PTFE) were procured from MatTek Corporation (Ashland, MA, USA). EpiCorneal COR-100 ver 2.0 3D tissue constructs (Lot#36849Kits A and G (used for permeability experiment) and Kits H and J (used for tissue accumulation experiment), medium (Cat. COR-100-MM), MTT concentrate (Cat. MTT-100-CON), MTT diluent (Cat. MTT-100-DIL), and extraction solution (Cat. MTT-100-EXT) were obtained from MatTek Life Sciences (Ashland, MA, USA). The tissues were shipped and stored at 4°C until use.

### 3.2. LC/MS-Method

### Liquid chromatography

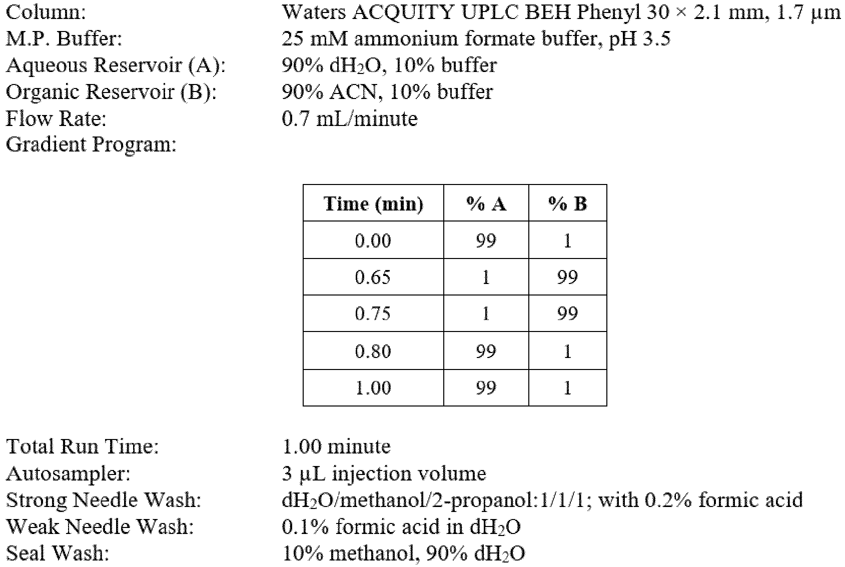

### Mass spectrometer

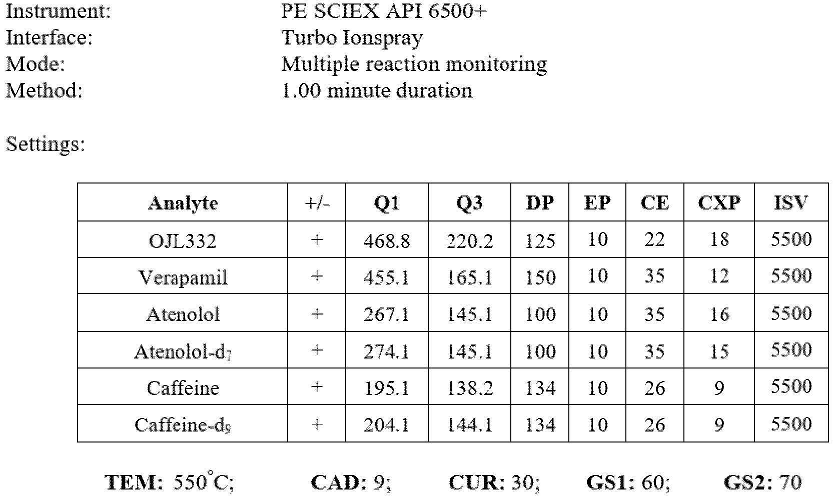

### 3.3. Unidirectional flux assessment across MatTek EpiCorneal tissues

Unidirectional flux through MatTek EpiCorneal tissues was evaluated for the aqueous ophthalmic composition Samples 1 to 6 of Example 1 in parallel with a positive control to disrupt the tissue integrity (1% TritonX-100) and a negative control (100 µM atenolol and 50 µM caffeine in PBS, pH 7.4).

Tissues were first equilibrated to 37°C overnight in MatTek medium according to MatTek protocol. The next day, the recovery incubation medium was removed. Permeability experiments were initiated by adding 110µL of dosing solution to the apical chamber for all treatments (100 µL for dose plus 10 µL for T=0 sampling). The basolateral chamber contained 500 µL of PBS, pH 7.4 with 1% BSA. Each formulation treatment was performed with six replicates (n=6) per treatment. The treatments for positive and negative controls were performed in triplicate (n=3). The tissues were incubated for 240 minutes in a humidified incubator at 37°C with 5% CO₂. Donor samples were collected at 0 and 240 minutes. Receiver samples (200 µL) were collected at 15, 30, 60, 120, 180, and 240 minutes and replaced with fresh buffer at each timepoint. All donor samples were diluted 2000-fold with 1% BSA in PBS, pH 7.4. The diluted donor samples and the undiluted receiver samples were then treated 2-fold with ACN (acetonitrile) in order to crash out the protein. The samples were mixed and centrifuged. The supernatants were then diluted 1:1 with dH₂O containing internal standard (0.2 µM verapamil) and analyzed using the LC-MS/MS method described in section 3.2. above.

At the end of the flux assessment incubation period, three replicates (n=3) from each sample group were rinsed with 300 µL of PBS, pH 7.4 and treated with tetrazolium salt (3-(4,5-dimethylthiazol-2-yl)- 2,5-diphenyltetrazolium bromide, MTT). The MTT solution was incubated on the tissues for 3 hours at 37°C in a humidified incubator with 5% CO₂. The MTT solution was removed, and the tissues were treated with extraction solution. The extraction took place at room temperature for 2 hours (protected from light). The final extraction solution was mixed and transferred to a 96-well plate. The samples were analyzed using a microplate reader at a wavelength of 570 nm. Three blank inserts (n=3) were treated similarly to the tissue samples and used as background correction for MTT calculations. The formulation-treated absorbance readings were compared to the negative control tissue absorbance and the % viability was calculated as follows: $% Viability = 100 \times \left[\left(OD \left(treated tissue\right)-OD \left(background tissues\right)/OD \left(negative control\right)\right.\right]$

The remaining tissue replicates (n=3 for Samples 1 to 6) were rinsed briefly with 300 µLPBS, pH 7.4 followed by aspiration of the wash, patted dry on a lab wipe, weighed, and stored in the freezer until tissue accumulation analysis was performed. Tissue samples were removed from the freezer and lysed with 19 volumes of75% ACN:25% PBS, pH 7.4. The mixture was allowed to sit for 30 minutes with shaking, centrifuged to pellet the undissolved tissue, and the supernatant was further diluted with 75% ACN:25% PBS, pH 7.4 (final dilution:1000-fold). The samples were mixed 1:1 with dH₂O containing IS (0.2 µM verapamil) and analyzed using the LC-MS/MS method described in section 3.2. above.

### 3.4. Exploratory assessment of tissue concentration in MatTek EpiCorneal tissues

Compound I accumulation in EpiCorneal tissues was evaluated for Samples 1 to 6 at two time points. Tissues were first equilibrated to 37°C overnight in MatTek medium according to MatTek protocol. The next day, the recovery incubation medium was removed. The experiment was initiated by adding 100 µL of each dosing solution to the apical chamber for all treatments. The basolateral chamber contained 500 µLof PBS, pH 7.4. Each treatment was performed with four replicates (n=4) per treatment per collection time. The tissues were incubated for the study duration (15 or 120 minutes) in a humidified incubator at 37°C with 5% CO₂.

At the end of the incubation duration, the four replicates (n=4) from each group were demounted, rinsed briefly with 300 µL PBS, pH 7.4 followed by aspiration of the wash, patted dry on a lab wipe, weighed, and stored in the freezer until tissue accumulation analysis was performed. Tissue samples were removed from the freezer and lysed with 19 volumes of 75% ACN:25% PBS, pH 7.4. The mixture was allowed to sit for 30 minutes with shaking, centrifuged to pellet the undissolved tissue, and the supernatant was further diluted with 75% ACN:25% PBS, pH 7.4 (final dilution: 1000-fold). The samples were prepared 1:1 with dH₂O containing IS (0.2 µM verapamil) and analyzed using the LC-MS/MS method described in section 3.2. above.

### 3.5. Data analysis

The cumulative concentration of each compound at each time point (C_{c}) was calculated as the sum of the measured concentration at that time point (Cₘ) and 200/500 of the measured concentrations at the previous time points (0.2 mL of total receiver sample at each time point out of the 0.5 mL total receiver volume was withdrawn and replaced at each timepoint).
For receiver samples at 15, 30, 60, 120, 180, and 240 minutes: ${C}_{c 15} = {C}_{m 15}$ ${C}_{c 30} = {C}_{m 30} + {C}_{m 15} \times 2 / 5$ ${C}_{c 60} = {C}_{m 60} + {C}_{m 30} + {C}_{m 15} \times 2 / 5$ ${C}_{c 120} = {C}_{m 120} + \left({C}_{m 15}+{C}_{m 30}+{C}_{m 60}\right) \times 2 / 5$ ${C}_{c 180} = {C}_{m 180} + \left({C}_{m 15}+{C}_{m 30}+{C}_{m 60}+{C}_{m 120}\right) \times 2 / 5$ ${C}_{c 240} = {C}_{m 240} + \left({C}_{m 15}+{C}_{m 30}+{C}_{m 60}+{C}_{m 120}+{C}_{m 180}\right) \times 2 / 5$
The Pₐₚₚ for Compound I, atenolol, and caffeine was calculated as follows: ${P}_{app} = \left(d{C}_{r}/dt\right) \times {V}_{r} / \left(A\times {C}_{N}\right)$ Where,
   *d*Cᵣ/*d*t is the slope of the cumulative receiver concentration (µM f or controls or µg/mL for Compound I) vs. time (t);
   Vᵣ is the volume of the receiver compartment (0.5 cm³);
   A is the exposed surface area for permeation (0.6 cm²);
   C_{N} is the nominal dosing concentration (µM for controls or µg/mL for test article).
The cumulative percent (%) of dose permeated for each compound was calculated as follows: $\left.% Permeated=100\times \left({C}_{r}\times {V}_{r}\right)/{D}_{N}\right)$ Where,
   Cᵣ is the cumulative receiver amount at each timepoint (µg);
   Vᵣ is the volume of the receiver compartment (0.5 mL);
   D_{N} is the nominal amount dosed (µg).
The flux for each compound was calculated as follows: $Flux = \left(d{C}_{r}/dt\right) \times {V}_{r} / A$ Where,
   *d*Cᵣ/*d*t is the slope of the cumulative receiver concentration (µg) vs. time (t);
   Vᵣ is the volume of the receiver compartment (0.5 mL);
   A is the exposed surface area for permeation (0.6 cm²).
Only samples collected in a linear range (meeting the linearity criterion of r 2≥ 0.9 for Cᵣ vs. t using the "RSQ" function in Microsoft Office 365 Excel) were used to calculate *d*Cᵣ/*d*t values. $Recovery = 100 \times \left(\left({V}_{r}\times {{C}_{r}}^{final}\right)+\left({{V}_{d}}^{initial}\times {{C}_{d}}^{0}\right)+\left({{V}_{d}}^{final}\times {{C}_{d}}^{final}\right)\right) / \left({V}_{d}\times {{C}_{d}}^{0}\right)$ Where,
   Vᵣ is the volume of the receiver compartment (0.5 mL);
   V_{d} is the volume of the donor compartment (0.110mL);
   V_{d}^{initial} is the volume sampled from the donor compartment (0.01 mL);
   V_{d}^{final} is the volume in the donor compartment (0.1mL);
   C_{d}⁰ is the measured donor concentration at 0 minutes (µg/mL);
   Cᵣ^{final} is the cumulative receiver concentration at the end of the incubation (µg/mL);
   C_{d}^{final} is the donor concentration at the end of the incubation (µg/mL).

### 3.6. Results

The average cumulative amount permeated for each Sample is displayed in Figure 1. The average cumulative amount permeated is presented as a percent of the dosed amount in Figure 2. The Pₐₚₚ and flux values of Compound I across EpiCorneal tissue are summarized in Table 2 and permeability is summarized in Figure 3.

**Table 2: Permeability of Compound I across MatTek EpiCorneal tissues (Mean, and SD (standard deviation) calculations were performed on n=6 replicates. The entire time course was used to calculate permeability and flux since the linear regression met the linearity criterion (r₂ ≥0.9)).**

| Sample | HPbCD:Compound I molar ratio | Pₐₚₚ [cm⁻⁶/s] | SD | Flux [µg/min/cm²] | SD |
|---|---|---|---|---|---|
| 1 | 13 | 0.194 | 0.028 | 0.0175 | 0.0025 |
| 2 | 14 | 0.177 | 0.043 | 0.0159 | 0.0039 |
| 3 | 22 | 0.094 | 0.014 | 0.0085 | 0.0013 |
| 4 | 35 | 0.122 | 0.011 | 0.0044 | 0.0004 |
| 5 | 106 | 0.119 | 0.019 | 0.0014 | 0.0002 |
| 8 | 163 | 0.111 | 0.048 | 0.0013 | 0.0006 |

The permeability results for the reference compounds, atenolol and caffeine, showed good separation (Pₐₚₚ ratio >>3), indicating good barrier integrity for the batch of tissues used in the study. The permeability for each treatment was less than or similar to that of the moderate permeability compound, atenolol. The viability results confirmed that the Sample formulations had no impact on the tissue viability, with all average results greater than 80% of the control.

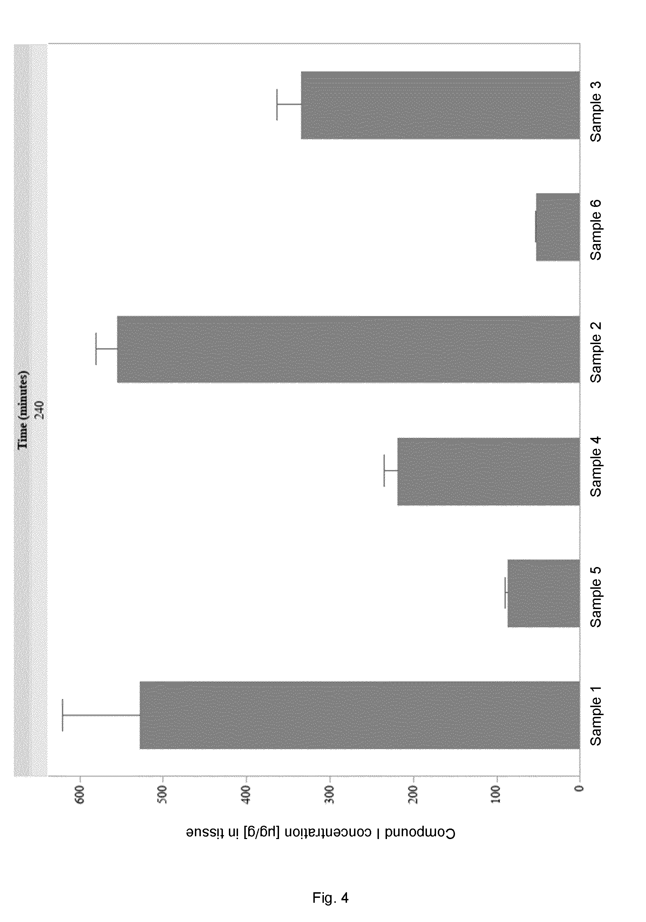

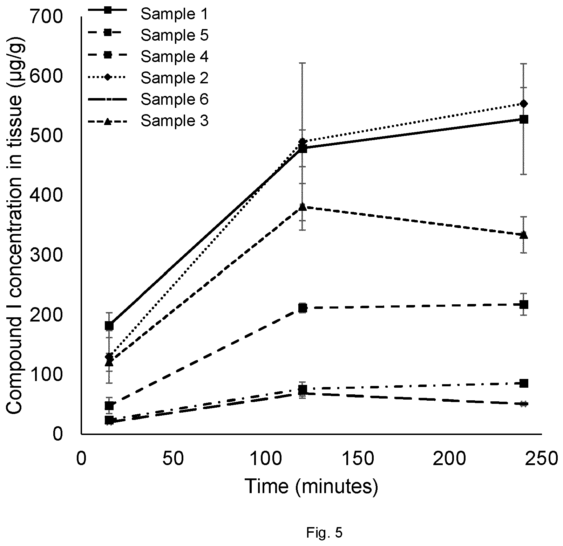

The tissue accumulation is presented Figure 4, and the time course of tissue accumulation of the exploratory tissue accumulation study is presented in Figure 5. The tissue accumulation was higher when dosed with a higher test article concentration. Tissue accumulation plateaued at 120 and 240 minutes for each dose concentration.

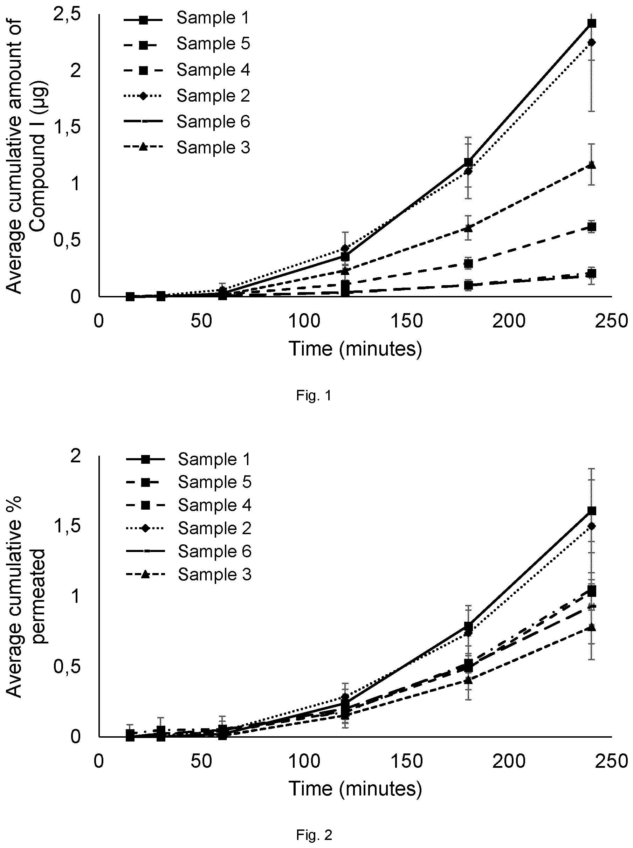

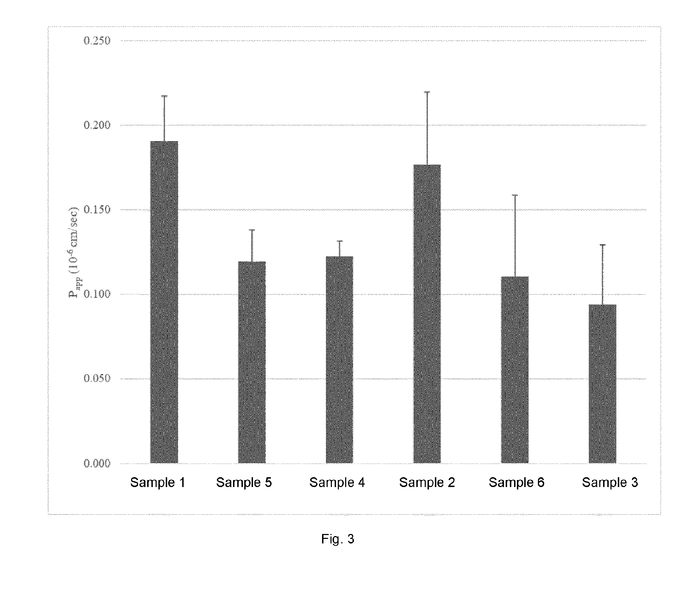

As can be seen from Figs. 1 to 5 and Table 2, the permeability of Compound I was significantly higher for the inventive samples 1 and 2. The same effect was observed for the Compound I concentration in the EpiCorneal tissue.

### 4. Example 3 - Preparation of aqueous ophthalmic compositions

Four aqueous ophthalmic compositions were prepared using the protocol described in Example 1, with the difference that NaCl was used as tonicity agent instead of mannitol. The compositions of the prepared aqueous ophthalmic formulations are listed in Table 3 below.

**Table 3: Composition and properties of aqueous ophthalmic compositions of Example 3.**

| **Sample** | **7** | **8** | **9** | **10** |
|---|---|---|---|---|
| Compound I | 3.0 mg/ml | 1.5 mg/ml | 0.6 mg/ml | 0.2 mg/ml |
| HPbCD | 129.0 mg/ml | 64.9 mg/ml | 26.0 mg/ml | 8.7 mg/ml |
| NaCl | 1.31 mg/ml | 3.13 mg/ml | 4.54 mg/ml | 5.05 mg/ml |
| Phosphate buffer (50 mM) | 7.8 mg/ml | 7.8 mg/ml | 7.8 mg/ml | 7.8 mg/ml |
| HCl | q.s. to pH 6.5 | q.s. to pH 6.5 | q.s. to pH 6.5 | q.s. to pH 6.5 |
| NaOH | q.s. to pH 6.5 | q.s. to pH 6.5 | q.s. to pH 6.5 | q.s. to pH 6.5 |
| Water for injection | q.s. to 1000 ml | q.s. to 1000 ml | q.s. to 1000 ml | q.s. to 1000 ml |
| HPbCD:Compound I molar ratio | 14:1 | 14:1 | 14:1 | 14:1 |
| HPbCD:Compound I concentration ratio | 43 | 43 | 43 | 44 |
| pH | 6.56 | 6.51 | 6.39 | 6.45 |
| Osmolality [mOsmol/kg] | 298 | 275 | 276 | 273 |

## Claims

1. An aqueous ophthalmic composition comprising:
1-(6-fluoro-5-((((1r,3r)-3-(4-fluoro-3(trifluoromethyl)phenoxy)-cyclobutyl)-amino)methyl)isoquinolin-8-yl)ethane-1.2-diol (Compound I) having structure 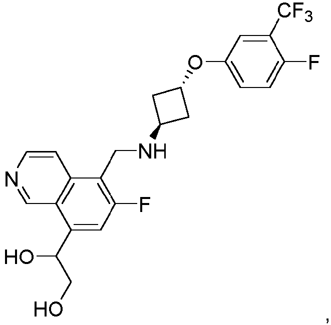 and
a cyclodextrin,
wherein the molar ratio of the cyclodextrin to the Compound I is from 5:1 to 19:1.

2. The aqueous ophthalmic composition of claim 1, wherein the cyclodextrin is selected from the group consisting of α-cyclodextrin, β-cyclodextrin, γ-cyclodextrins, derivatives thereof, and mixtures thereof, preferably the cyclodextrin is selected from the group consisting of α-cyclodextrin, β-cyclodextrin, methyl-β-cyclodextrin, 2-hydroxypropyl-β-cyclodextrin, 2-hydroxyethyl-β-cyclodextrin, sulfobutylether-β-cyclodextrin, heptakis (2,6-di-*O*-methyl)-β-cyclodextrin, heptakis (2,3,6-tri-*O*-methyl)-β-cyclodextrin, succinyl-β-cyclodextrin, 6-*O*-α-Maltosyl-β-cyclodextrin, 2-hydroxypropyl-γ-cyclodextrin, 2-hydroxyethyl-γ-cyclodextrin, and mixtures thereof, more preferably the cyclodextrin is selected from the group consisting of β-cyclodextrin, , methyl-β-cyclodextrin, 2-hydroxypropyl-β-cyclodextrin, 2-hydroxyethyl-β-cyclodextrin, sulfobutylether-β-cyclodextrin, heptakis (2,6-di-*O*-methyl)-β-cyclodextrin, heptakis (2, 3, 6-tri-*O*-methyl)-β-cyclodextrin, succinyl-β-cyclodextrin, 6-*O*-α-Maltosyl-β-cyclodextrin, and mixtures thereof, and most preferably the cyclodextrin is 2-hydroxypropyl-β-cyclodextrin.

3. The aqueous ophthalmic composition of any one of the preceding claims, wherein the composition comprises:
the Compound I in an amount from 0.01 to 1 wt.-%, preferably from 0.02 to 0.7 wt.-%, more preferably from 0.04 to 0.5 wt.-%, and most preferably from 0.06 to 0.3 wt.-%, based on the total weight of the composition, and/or
the cyclodextrin in an amount from 0.01 to 50 wt.-%, preferably from 0.05 to 40 wt.-%, more preferably from 0.07 to 30 wt.-%, and most preferably from 0.1 to 20 wt.-%, based on the total weight of the composition, and/or
wherein the molar ratio of the cyclodextrin to the Compound I is from 8:1 to 18:1, preferably from 10:1 to 17:1, more preferably from 11:1 to 16:1, even more preferably from 12:1 to 15:1, still even more preferably from 12.5 to 15:1, and most preferably from 13:1 to 14:1.

4. The aqueous ophthalmic composition of any one of the preceding claims, wherein the composition comprises a tonicity agent, preferably the tonicity agent is selected from the group consisting of salts, non-electrolytes, and mixtures thereof, more preferably the tonicity agent is selected from the group consisting of boric acid, inorganic salts, polyols, dextrose, trehalose, urea, and mixtures thereof, even more preferably the tonicity agent is selected from the group consisting of boric acid, sodium chloride, potassium chloride, magnesium chloride, calcium chloride, sodium sulphate, sodium hydrogen phosphate, trisodium citrate, trisodium phosphate, magnesium sulphate, zinc sulphate, sodium carbonate, mannitol, glucose, glycerol, xylitol, sorbitol, propylene glycol, dextrose, trehalose, urea, and combinations thereof, and most preferably the tonicity agent is selected from the group consisting of sodium chloride, potassium chloride, magnesium chloride, mannitol, and mixtures thereof.

5. The aqueous ophthalmic composition of claim 4, wherein the composition comprises the tonicity agent in an amount such that the composition exhibits an osmolality of from 100 to 500 mOsmol/kg, preferably from 225 to 430 mOsmol/kg, and most preferably from 250 to 350 mOsmol/kg.

6. The aqueous ophthalmic composition of any one of the preceding claims, wherein the composition comprises a buffer, preferably the buffer is selected from the group consisting of phosphate buffer, phosphate citrate buffer, citrate buffer, tartrate buffer, borate buffer, malate buffer, succinate buffer, acetate buffer, acetate borate buffer, MES (2-(N-morpholino)ethanesulfonic acid), HEPES (2-(4-(2-hydroxyethy1)I-piperazinyl-ethanesulfonic acid), BES (N,N-bis(2 -hydroxyethyl)-2-aminoethanesulfonic acid), MOPS (3-(N-morpholino)propanesulfonic acid), TRIS (tris(hydroxymethyl)amino-methane), and BIS-TRIS (bis(2-hydroxyethyl)amino-tris(hydroxymethyl)methane, more preferably the buffer is selected from the group consisting of phosphate buffer, phosphate citrate buffer, citrate buffer, borate buffer, or TRIS, and most preferably the buffer is a citrate buffer.

7. The aqueous ophthalmic composition of any one of the preceding claims, wherein the pH of the composition is from 5.0 to 8.0, preferably from 5.5 to 7.9, more preferably 6.0 to 7.8, even more preferably from 6.2 to 7.5, and most preferably about 6.5 to 7.4.

8. The aqueous ophthalmic composition of any one of the preceding claims, wherein the composition further comprises a preservative, preferably the preservative is selected from the group consisting of cetrimide, benzododecinium chloride, benzalkonium chloride, thiomersal, alexidine, polyquad, hydrogen peroxide, sodium hydroxymethylglycinate, polyhexanide, sorbic acid, sodium perborate, oxyd (stabilized sodium chlorite with hydrogen peroxide), SofZia^{®} (preservative system composed of boric acid, propylene glycol, sorbitol, and zinc chloride), and mixtures thereof, and most preferably the preservative is cetrimide, benzalkonium chloride, or a mixture thereof.

9. The aqueous ophthalmic composition of any one of the preceding claims, wherein the composition comprises:
0.01 to 0.5 wt.-%, preferably 0.02 to 0.3 wt.-%, based on the total weight of the composition, of the Compound I,
1 to 30 wt.-%, preferably 2 to 20 wt.-%, based on the total weight of the composition, of the cyclodextrin, preferably 2-hydroxypropyl-β-cyclodextrin,
optionally 0.7 to 1.2 wt.-%, based on the total weight of the composition, of a buffer, preferably a phosphate buffer or citrate buffer,
optionally 0.1 to 7 wt.-%, preferably 0.2 to 5 wt.-%, based on the total weight of the composition, of a tonicity agent, preferably sodium chloride or mannitol,
sodium hydroxide and/or hydrochloric acid for pH adjustment, and
q.s. ad 100 wt.-% water.

10. The aqueous ophthalmic composition of any one of the preceding claims, wherein
the composition, at a shear rate of 10 s⁻¹, exhibits a viscosity of less than 350 mPa·s at 35°C, preferably less than 250 mPa·s at 35°C, more preferably less than 200 mPa·s at 35°C, even more preferably less 150 mPa·s at 35°C, and most preferably less than 100 mPa·s at 35°C, and/or
the composition exhibits an osmolality of from 100 to 500 mOsmol/kg, preferably from 225 to 430 mOsmol/kg, and most preferably from 250 to 350 mOsmol/kg.

11. The composition of any one of the preceding claims, wherein the composition is in form of an eye lid spray, an eye bath, an eye wash solution, or eye drops.

12. A method for preparing an aqueous ophthalmic composition according to any one of the preceding claims, wherein the method comprises the steps of:
mixing the Compound I, the cyclodextrin, and water, and
adjusting the pH to a range from 5.0 to 8.0.

13. An aqueous ophthalmic composition according to any one of claims 1 to 11 for use as a medicament.

14. An aqueous ophthalmic composition according to any one of claims 1 to 11, for use in treating a disease or a condition of the eye or the organ or tissue surrounding the eye or being connected therewith, preferably for use in treating ocular surface disorders, more preferably for use in treating ocular surface pain, dry eye disease, Sjogren's Syndrome, conjunctivitis, Map-Dot-Fingerprint Dystrophy, acanthamoeba, fibromyalgia, Meibomian gland dysfunction, thyroid eye disease, rosacea, ptosis, keratoconus, ocular pain syndrome, Steven-Johnson's syndrome, corneal epitheliopathies, corneal neuropathies, corneal dystrophies, epithelial basement membrane dystrophy, corneal erosions or abrasions, ocular surface diseases, blepharitis, graft vs. host disease, meibomitis, glaucoma, conjunctivochalasis, keratopathis, keratitis, iritis, episclentis, corneal surgery, multiple sclerosis, trichiasis, pterygium, neuralgia, xerophthalmia, or patients recovering from neurotrophic keratitis, and most preferably for use in treating ocular surface pain.

15. A container comprising the aqueous ophthalmic composition according to any one of claims 1 to 11, wherein the container is a single-dose container, preferably a single-dose ophtiole, or a multi-dose container, preferably an ophtiole, or an application system, preferably a tip-seal application system, a pump-based application system, a standard bottle/dropper system, or a multi-dose application system.
